(19) Europäisches Patentamt
European Patent Office
Office européen des brevets

(11) Veröffentlichungsnummer: **0 293 714 B1**

# (12) EUROPÄISCHE PATENTSCHRIFT

(45) Veröffentlichungstag der Patentschrift: **05.06.91 Patentblatt 91/23**

(51) Int. Cl.$^5$: **A61K 31/44**, A61K 31/505

(21) Anmeldenummer: **88108234.1**

(22) Anmeldetag: **24.05.88**

## (54) Neue Kombinationspräparate mit antidepressiver Wirkung.

(30) Priorität: **02.06.87 DE 3718398**

(43) Veröffentlichungstag der Anmeldung: **07.12.88 Patentblatt 88/49**

(45) Bekanntmachung des Hinweises auf die Patenterteilung: **05.06.91 Patentblatt 91/23**

(84) Benannte Vertragsstaaten: **AT BE CH DE ES FR GB IT LI NL SE**

(56) Entgegenhaltungen:
**DIALOG 6425109, Embase no. 87161816: E. MOGILNICKA et al.: "Dihydropyridine calcium channel antagonists reduce immobility in the mouse behavioral despair test; antidepressants facilitate nifedipine action", & EUR. J. PHARMACOL. 1987, 138/3 (413-416)**

(73) Patentinhaber: **Troponwerke GmbH & Co. KG
Berliner Strasse 156
W-5000 Köln 80 (DE)**

(72) Erfinder: **Traber, Jörg, Dr.
Löwenburgstrasse 12
W-5204 Lohmar 21 (DE)**
Erfinder: **Horstmann, Harald, Dr.
Claudiusweg 19
W-5600 Wuppertal 1 (DE)**

(74) Vertreter: **Mann, Volker, Dr. et al
c/o Bayer AG Konzernverwaltung RP
Patentabteilung
W-5090 Leverkusen 1, Bayerwerk (DE)**

EP 0 293 714 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft antidepressiv wirksame Kombinationspräparate mit synergistischer Wirkung enthaltend antidepressiv wirksame Stoffe und Calcium-antagonistisch wirksame Verbindungen aus der Klasse der Dihydropyridine, sowie die Verwendung der Dihydropyridine zur Herstellung von Arzneimitteln zur Behandlung von Depressionen.

Aus dem Stand der Technik sind eine Vielzahl von antidepressiv wirksamen Stoffen bekannt, wie z.B. Dibenzocycloheptene und verwandte Strukturklassen der tricyclischen Antidepressiva (vgl. z.B. US-Patente 3 438 981, 3 420 851, 3 534 041, Belgisches Patent 61591, J. Org. Chem. 27, 4134 (1962), US-Patent 3 505 321 und Britisches Patent 858 187 und 858 188). Auch Verbindungen aus den Stoffklassen der tetracyclischen Antidepressiva, der Monoaminoxidase (MAO)-Hemmer, der basischen Oximether und andere werden medizinisch als Antidepressiva eingesetzt (vgl. Oxaprotilin, Mianserin, Tranylcypromin, Pargylin, Zimelidin, Fluvoxamin und Trazodon).

Die Dihydropyridine mit kalciumantagonistischer Wirkung sind ebenfalls weitgehend bekannt (vgl. Britisches Patent 1 173 862, 1 358 951, US-Patent 4 256 749 und 4 264 611). Für diese Dihydropyridine sind bereits eine Reihe von pharmakologischen Wirkungen beschrieben wie z.B. Coronarwirkung, Blutdruckwirkung, diuretische Wirkung oder antiischämische Wirkung im cerebralen Bereich. Eine direkte antidepressive Wirkung oder eine solche verstärkende Wirkung ist für Dihydropyridine bisher noch nicht bekannt geworden.

Für einige Kalciumantagonisten, wie z.B. Verapamil, existieren Literaturhinweise für ihre Verwendung bei der Behandlung von manischen Patienten (vgl. Dubovsky SL et al (1982) ; Effectiveness of verapamil in the treatment of a manic patient, Am. J. Psychiatry 139 ; 502-504). Cerebrale und neurologische Wirkungen sind für Dihydropyridine, Nimodipin und Nicardipin bereits beschrieben worden (vgl. F. Hoffmeister et al (1982) Arznei-Forsch. 44 : 347-360). Nicardipin wurde auch auf eine Verwendung als antimanisch wirksamer Stoff geprüft (vgl. N. Renwart et al, Prog. Neuro-Psychopharmacol.- Biol. Psychiat. 1986, Vol. 10 ; 717-722). Hierbei zeigten einige Unterschiede in der Wirkung von Dihydropyridinen unter anderen Kalciumantagonisten wie Verapamil oder Diltiazem. Als Ergebnis dieser Untersuchungen zeigte sich, daß Kalciumantagonisten nicht geeignet sind als antimanische Wirkstoffe.

Bei einer Reihe von bisher bekannten antidepressiv wirksamen Stoffen können unerwünschte Nebenwirkungen auftreten, insbesondere wenn diese Wirkstoffe in hohen Dosierungen verabreicht werden. Weiterhin treten häufig Schwierigkeiten bei der galenischen Zubereitung auf, wenn Wirkstoffe mit höherer Dosierung in Applikationsformen überführt werden müssen, die vom Patienten ohne Mühe eingenommen werden sollen, z.B. bei oraler Verabreichung werden kleinere Tabletten bevorzugt. Generell ist es vom medizinischen Standpunkt aus erwünscht, die Wirkstoffdosierung so niedrig wie möglich zu halten. Ein weiterer Nachteil einiger bekannter Antidepressiva ist der relativ späte Wirkungseintritt, der häufig erst einige Tage nach Erstapplikation in zufriedenstellender Weise zu beobachten ist. Durch eine Wirkungsverstärkung bei gleichzeitiger Reduzierung der Wirkstoffmenge, kann ein früheres Eintreten des gewünschten Effektes erreicht werden.

Aufgabe der vorliegenden Erfindung ist das zur Verfügungstellen von antidepressien Mitteln mit geringem bzw. reduziertem Wirkstoffgehalt und damit vermindertem Nebenwirkungsrisiko. Diese Aufgabe kann durch die erfindungsgemäße Kombination der antidepressiven Wirkstoffe mit bestimmten Dihydropyridinen mit claciumantagonistischer Wirkung gelöst werden.

Die Erfindung betrifft die Verwendung von Dihydropyridinen der allgemeinen Formel (I)

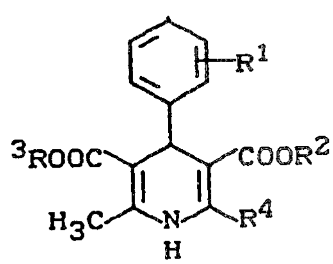

(I)

in welcher

$R^1$ für ein oder zwei gleiche oder verschiedene Substituenten aus der Gruppe Nitro, Halogen, Trifluormethyl, $OCHF_2$ oder für den Rest =N-O-N= steht, der an den Phenylring kondensiert ist,

$R^2$ und $R^3$ gleich oder verschieden sind und jeweils für Alkyl mit 1 bis 12 Kohlenstoffatomen stehen, welches gegebenenfalls substituiert ist durch Alkoxy mit 1 bis 4 C-Atomen, Hydroxy, Halogen oder N-Methyl-N-Benzylamino

und
$R^4$ für Cyano oder Alkyl mit 1 bis 4 Kohlenstoffatomen steht, welches gegebenenfalls substituiert ist durch Hydroxy oder Halogen,
zur Herstellung von Arzneimitteln zur Behandlung von Depressionen, insbesondere zur Verstärkung der antidepressiven Wirkung in Kombinationspräparaten mit antidepressiv wirksamen Verbindungen aus der Gruppe Nortriptylin, Amitriptylin, Imipramin, Desipramin, Doxepin, Citalopram, Fluoxetin, Fluvoxamin, Mianserin, Oxaprotilin und Maprotilin.
Von besonderer Bedeutung sind Dihydropyridine aus der Gruppe Nifedipin, Niludipin, Nisoldipin, Nitrendipin, Nimodipin, Felodipin, Nicardipin.

Überraschenderweise wird dieser überadditiv wirkungsverstärkende Effekt nur durch die Verwendung von Kalciumantagonisten aus der Klasse der genannten Dihydropyridine erreicht. Kalciumantagonisten aus anderen Stoffklassen wie z.B. Verapamil oder Diltiazem zeigen diesen Verstärkungseffekt nicht.

Die folgenden Versuche belegen beispielhaft den unerwarteten, überadditiven synergistischen Effekt durch Kombination einiger exemplarischer Antidepressiva mit kalciumantagonistisch wirksamen Dihydropyridinen.

Testmethode

Männliche Albinomäuse, die unter Standardlaborbedingungen mit freiem Zugang zu Futter und Wasser gehalten wurden, werden für 6 Minuten in einen mit Wasser gefüllten Zylinder gebracht. Die Mäuse geben nach einiger Zeit den Versuch, dem Wasser zu entkommen auf und verfallen in eine Immobilität. Die Dauer der Immobilität während der letzten 4 Minuten wird gemessen. Bei unbehandelten Kontrollgruppen beträgt die Dauer der Immobilität 2 bis 3 Minuten. Viele Antidepressiva verkürzen die Dauer der Immobilität, wenn sie der Maus in relativ hoher Dosis (ca. 30 mg/kg) appliziert werden, während dieser Effekt nach niedriger Dosierung nicht beobachtet wird. Überraschenderweise verkürzt sich die Immobilitätsphase nach Applikation der erfindungsgemäßen Kombination mit Dihydropyridinen jedoch auch nach niedriger Dosierung (Beschreibung der Methode : R.B. Porsolt et al., Biochem. Pharmacol. 34, 3837 (1977)).

Die folgende Tabelle 1 zeigt die Ergebnisse nach einfacher und nach kombinierter Applikation der jeweiligen verschiedenen Wirkstoffe.

Tabelle 1 :

Wirkung bei separater und bei gleichzeitiger Applikation von Nifedipin und verschiedenen Antidepressiva auf die Dauer der Immobilität von Mäusen während der letzten 4 Minuten des 6 Minuten Wassertestes. (Jeweils Mittelwerte von 10 Tieren pro Versuch).

| Behandlungsart (Wirkstoff) | *Dosis (mg/kg) | Dauer der Immobilität (s) Mittelwert (± S.E.M.) |
|---|---|---|
| Kontrolle | - | 157.3 (9.1) |
| Imipramin | 10 | 174.6 (12.2) |
| Mianserin | 10 | 184.4 (14.0) |
| Citalopram | 10 | 192.6 (16.3) |
| | | |
| Kontrolle | - | 157.3 (9.1) |
| Nifedipin + Imipramin | 20 + 10 | 24.2 (5.1) |
| Nifedipin + Mianserin | 20 + 20 | 20.6 (3.2) |
| Nifedipin + Citalopram | 20 + 10 | 23.3 (7.4) |
| | | |
| Kontrolle | - | 155.2 (12.4) |
| (+)Oxaprotilin | 5 | 111.0 (6.7) |
| Nifedipin + (+)Oxaprotilin | 20 + 5 | 21.1 (3.5) |
| | | |
| Kontrolle | - | 163.4 (13.3) |
| Nifedipin | 20 | 101.3 (10.4) |
| (-)Oxaprotilin | 30 | 130.7 (12.1) |
| Nifedipin + (-)Oxaprotilin | 20 + 20 | 67.8 (6.8) |

* Die Application erfolgt für die Antidepressiva intraperitoneal und für die Dihydropyridine oral ; jeweils 30 Minuten vor Testbeginn.

Tabelle 2 zeigt die antidepressive Wirkung einiger beispielhafter Dihydropyridine im Vergleich zur fehlenden Wirkung bei Kalciumantagonisten aus anderen Stoffklassen wie z.B. Verapamil oder Diltiazem.

Tabelle 2:

| Behandlungsart (Wirkstoff) | Dosis (mg/kg) | Dauer der Immobilität (s) Mittelwert (± S.E.M.) |
|---|---|---|
| Dihydropyridine | | |
| Kontrolle | - | 151.6 (9.4) |
| Nifedipin | 0.1 | 147.0 (9.5) |
| | 1.0 | 79.1 (10.2) |
| | 10.0 | 42.7 (4.4) |
| Kontrolle | - | 126.6 (9.9) |
| Nitrendipin | 0.1 | 99.2 (11.2) |
| | 1.0 | 62.4 (4.6) |
| | 10.0 | 44.0 (4.1) |
| Phenylalkylamine | | |
| Kontrolle | - | 165.5 (8.3) |
| Verapamil | 0.1 | 142.1 (9.7) |
| | 1.0 | 180.5 (7.0) |
| | 10.0 | 163.5 (12.3) |
| Benzothiazepine | | |
| Kontrolle | - | 148.1 (8.3) |
| Diltiazem | 0.1 | 135.3 (12.6) |
| | 1.0 | 142.1 (10.0) |
| | 10.0 | 137.9 (11.5) |

Besonders geeignet sind erfindungsgemäße Kombinationspräparate deren Tagedosis für die antidepressiven Wirkstoffe 10 bis 200 mg, insbesondere 20 bis 100 mg und für die kalciumantagonistisch wirksamen Dihydropyridine 5 bis 100 mg, insbesondere 10 bis 50 mg beträgt. Diese Wirkstoffmengen können als Einmaldosierung verabreicht oder über mehrere Dosierungen pro Tag verteilt werden.

Die erfindungsgemäßen Kombinationspräparate können in üblichen galenischen Formulierungen eingesetzt werden, wie Tabletten, Kapseln, Dragees, Granulate, Sirupe, Emulsionen, Suspensionen und Lösungen, unter Verwendung inerter, pharmazeutisch geeigneter Trägerstoffe und Lösungsmittel. Hierbei sollen die therapeutisch wirksamen Verbindungen jeweils in einer Konzentration von etwa 1 bis 90 Gew.-% der Gesamtmischung vorhanden sein, d.h. in Mengen die ausreichend sind, um den angegebenen Dosierungsspielraum zu erreichen.

Die Formulierungen werden beispeilsweise hergestellt durch Verstreckungen der Wirkstoffe mit Lösungsmittel und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt :
Wasser, nicht-toxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürlich Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und/oder dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendung gedacht sind, können die Wirkstoffe außer den obengenannten Hilfsstoffen mit verschiedenen Geschmackaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 0,5 mg/kg, vorzugsweise etwa 0,01 bis 0,2 mg/kg Körpergewicht zur Erzielung wirksamer Ergebnisse zu verabreichen, und bei oraler Applikation beträgt die Dosierung etwa 0,05 bis 5 mg/kg, vorzugsweise 0,1 bis 3 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierfür auch die obigen Ausführungen.

## Ansprüche

**Patentsprüche für die Vertragsstaaten : AT, BE, CH, DE, FR, GB, IT, LI, NL, SE**

1. Verwendung von kalciumantagonistisch wirksamen Dihydropyridinen der allgemeinen Formel

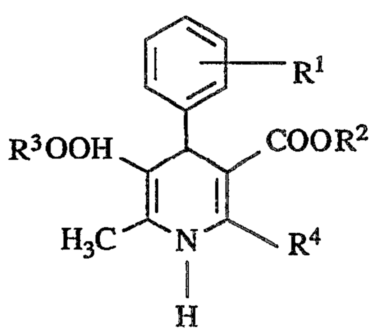

(I)

in welcher

$R^1$ für ein oder zwei gleiche oder verschiedene Substituenten aus der Gruppe Nitro, Halogen, Trifluormethyl, Difluormethoxy oder für den Rest

=N-O-N=

steht, der an den Phenylring kondensiert ist,

$R^2$ und $R^3$ gleich oder verschieden sind und jeweils für Alkyl mit 1 bis 12 Kohlenstoffatomen stehen,welches gegebenenfalls substituiert ist durch Alkoxy mit 1 bis 4 Kohlenstoffatomen, Hydroxy, Halogen oder N-Methyl-N-Benzylamino

und

$R^4$ für Cyano oder Alkyl mit 1 bis 4 Kohlenstoffatomen steht, welches gegebenenfalls substituiert ist durch Hydroxy oder Halogen, zur Herstellung von Arzneimitteln zur Behandlung von Depressionen.

2. Antidepressive Kombinationspräparate mit überadditiv verstärkter Wirkung, enthaltend einen antidepressiven Wirkstoff aus der Gruppe Nortriptylin, Amitriptylin, Imipramin, Desipramin, Citalopram, Fluoxetin, Fluvoxamin, Mianserin, Oxaprotilin und Maprotilin und ein Dihydropyridin der Formel (I) gemäß Anspruch 1.

3. Verfahren zur Herstellung von antidepressiven Kombinahonspräparaten mit überaddihv verstärkter Wirkung, enthaltend einen antidepressiven Wirkstoff aus der Gruppe Nortriptylin, Amitriptylin, Imipramin, Desipramin, Citalopram, Fluoxetin, Fluvoxamin, Mianserin, Oxaprotilin und Maprotilin und ein kalciumantagonistisch wirksames Dihydropyridin der allgemeinen Formel

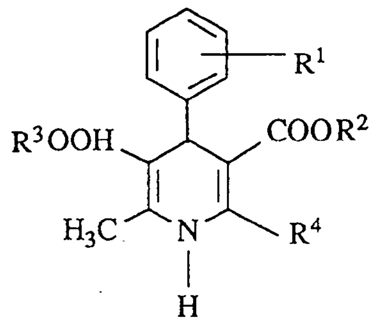

(I)

in welcher

$R^1$ für ein oder zwei gleiche oder verschiedene Substituenten aus der Gruppe Nitro, Halogen, Trifluormethyl, Difluormethoxy oder für den Rest

=N-O-N=

steht, der an den Phenylring kondensiert ist,

$R^2$ und $R^3$ gleich oder verschieden sind und jeweils für Alkyl mit 1 bis 12 Kohlenstoffatomen stehen,welches gegebenenfalls substituiert ist durch Alkoxy mit 1 bis 4 Kohlenstoffatomen, Hydroxy, Halogen oder N-Methyl-N-Benzylamino

und

$R^4$ für Cyano oder Alkyl mit 1 bis 4 Kohlenstoffatomen steht, welches gegebenenfalls substituiert ist durch Hydroxy oder Halogen, dadurch gekennzeichnet, daß man die Dihydropyridine und die antidepressiver Wirkstoffe, gegebenenfalls unter Verwendung von üblichen Hilfs- und Trägerstoffen, in eine geeignete Applikationsform überführt.

**Patentansprüche für den Vertragsstaat : ES**

1. Verfahren zur Herstellung von antidepressiven Kombinationspräparaten mit überadditiv verstärkter Wirkung, enthaltend einen antidepressiven Wirkstoff aus der Gruppe Nortriptylin, Amitriptylin, Imipramin, Desipramin, Citalopram, Fluoxetin, Fluvoxamin, Mianserin, Oxaprotilin und Maprotilin und ein kalciumantagonistisch wirksames Dihydropyridin der allgemeinen Formel

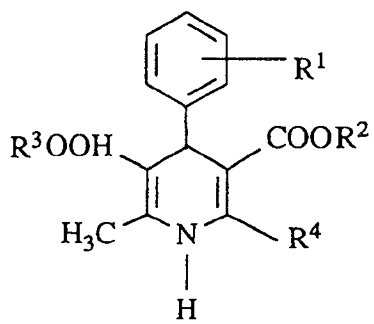

(I)

in welcher

$R^1$ für ein oder zwei gleiche oder verschiedene Substituenten aus der Gruppe Nitro, Halogen, Trifluormethyl, Difluormethoxy oder für den Rest

=N-O-N=

steht, der an den Phenylring kondensiert ist,

$R^2$ und $R^3$ gleich oder verschieden sind und jeweils für Alkyl mit 1 bis 12 Kohlenstoffatomen stehen, welches gegebenenfalls substituiert ist durch Alkoxy mit 1 bis 4 Kohlenstoffatomen, Hydroxy, Halogen oder N-Methyl-N-Benzylamino

und

$R^4$ für Cyano oder Alkyl mit 1 bis 4 Kohlenstoffatomen steht, welches gegebenenfalls substituiert ist durch Hydroxy oder Halogen, dadurch gekennzeichnet, daß man die Dihydropyridine und die antidepressiven Wirkstoffe, gegebenenfalls unter Verwendung von üblichen Hilfs und Trägerstoffen, in eine geeignete Applikationsform überführt.

2. Verfahren nach Anspruch 1, dadurch gekennzeichnet, daß als Kalciumantagonistisches Dihydropyridin Nifedipin, Niludipin, Nisoldipin, Nitrendipin, Nimodipin, Felodipin oder Nicardipin eingesetzt wird.

3. Verfahren nach Anspruch 1, wobei der antidepressive Wirkstoff in einer Menge von 10 bis 200 mg und das calciumantagonistisch wirksame Dihydropyridin in einer Menge von 5 bis 100 mg im Arzneimittel vorhanden ist.

4. Verfahren zur Herstellung von Arzneimitteln, enthaltend calciumantagonistische Dihydropyridine der allgemeinen Formel

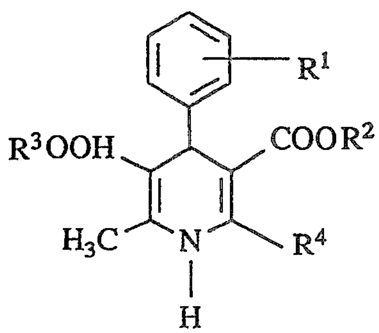

(I)

in welcher

$R^1$ für ein oder zwei gleiche oder verschiedene Substituenten aus der Gruppe Nitro, Halogen, Trifluormethyl, Difluormethoxy oder für den Rest

=N-O-N=

steht, der an den Phenylring kondensiert ist,

$R^2$ und $R^3$ gleich oder verschieden sind und jeweils für Alkyl mit 1 bis 12 Kohlenstoffatomen stehen, welches gegebenenfalls substituiert ist durch Alkoxy mit 1 bis 4 Kohlenstoffatomen, Hydroxy, Halogen oder N-Methyl-N-Benzylamino

und

$R^4$ für Cyano oder Alkyl mit 1 bis 4 Kohlenstoffatomen steht, welches gegebenenfalls substituiert ist durch Hydroxy oder Halogen zur Behandlung von Depressionen, dadurch gekennzeichnet, daß man die Dihydropyridine unter Verwendung von üblichen Hilfsund Trägerstoffen in eine geeignete Applikationsform überführt.

## Claims

**Claims for the contracting States : AT, BE, CH, DE, FR, GB, IT, LI, NL, SE**

1. Use of dihydropyridines having calcium-antagonistic activity and the general formula

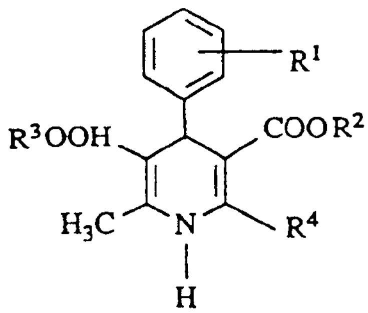

(I)

in which

$R^1$ represents one or two identical or different substituents from the group comprising nitro, halogen, trifluoromethyl or difluoromethoxy, or represents the radical

=N-O-N=,

which is fused onto the phenyl ring

$R^2$ and $R^3$ are identical or different and each represents alkyl which has 1 to 12 carbon atoms and is optionally substituted by alkoxy having 1 to 4 carbon atoms, hydroxyl, halogen or N-methyl-N-benzylamino, and

$R^4$ represents cyano or alkyl which has 1 to 4 carbon atoms and is optionally substituted by hydroxyl or halogen, for the preparation of medicaments for treating depression.

2. Antidepressant combination products having a superadditively enhanced action, containing an antidepressant active compound from the group comprising nortriptyline, amitriptyline, imipramine, desipramine, citalopram, fluoxetine, fluvoxamine, mianserin, oxaprotiline and maprotiline and a dihydropyridine of the formula (I) according to Claim 1.

3. Process for the preparation of antidepressant combination products having a superadditively enhanced action containing an antidepressant active compound from the group comprising nortriptyline, amitriptyline, imipramine, desipramine, citalopram, fluoxetine, fluvoxamine, mianserin, oxaprotiline and maprotiline and a dihydropyridine having calcium-antagonistic activity and the general formula

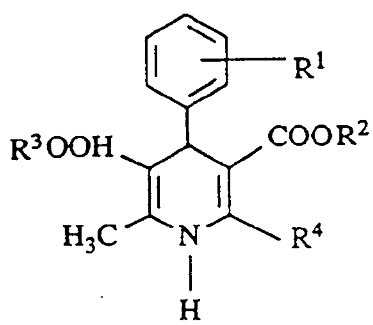

(I)

in which

$R^1$ represents one or two identical or different substituents from the group comprising nitro, halogen, trifluoromethyl or difluoromethoxy, or represents the radical

=N-O-N=,

which is fused onto the phenyl ring

$R^2$ and $R^3$ are identical or different and each represents alkyl which has 1 to 12 carbon atoms and is optionally substituted by alkoxy having 1 to 4 carbon atoms, hydroxyl, halogen or N-methyl-N-benzylamino, and

$R^4$ represents cyano or alkyl which has 1 to 4 carbon atoms and is optionally substituted by hydroxyl or halogen, characterised in that the dihydropyridines and the antidepressant active compounds are converted, where appropriate using customary auxiliaries and vehicles, into a suitable administration form.

**Claims for the contracting State : ES**

1. Process for the preparation of antidepressant combination products having a superadditively enhanced

action containing an antidepressant active compound from the group comprising nortriptyline, amitriptyline, imipramine, desipramine, citalopram, fluoxetine, fluvoxamine, mianserin, oxaprotiline and maprotiline and a dihydropyridine having calcium-antagonistic activity and the general formula

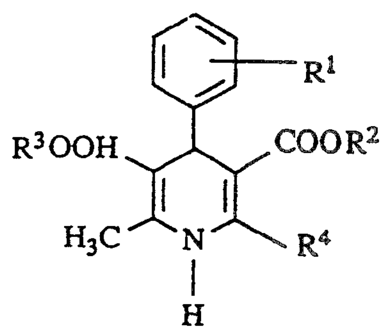

(I)

in which

$R^1$ represents one or two identical or different substituents from the group comprising nitro, halogen, trifluoromethyl or difluoromethoxy, or represents the radical

=N-O-N=,

which is fused onto the phenyl ring

$R^2$ and $R^3$ are identical or different and each represents alkyl which has 1 to 12 carbon atoms and is optionally substituted by alkoxy having 1 to 4 carbon atoms, hydroxyl, halogen or N-methyl-N-benzylamino, and

$R^4$ represents cyano or alkyl which has 1 to 4 carbon atoms and is optionally substituted by hydroxyl or halogen, characterised in that the dihydropyridines and the antidepressant active compounds are converted, where appropriate using customary auxiliaries and vehicles, into a suitable administration form.

2. Process according to Claim 1, characterised in that nifedipine, niludipine, nisoldipine, nitrendipine, nimodipine, felodipine or nicardipine is employed as calcium-antagonistic dihydropyridine.

3. Process according to Claim 1, where the amount of antidepressant active compound present in the medicament is 10 to 200 mg and the amount of dihydropyridine having calcium-antagonistic activity is 5 to 100 mg.

4. Process for the preparation of medicaments containing calcium-antagonistic dihydropyridines of the general formula

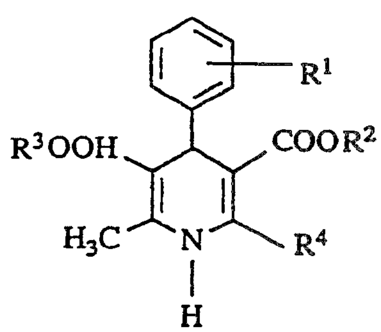

(I)

in which

$R^1$ represents one or two identical or different substituents from the group comprising nitro, halogen, trifluoromethyl or difluoromethoxy, or represents the radical

=N-O-N=,

which is fused onto the phenyl ring

$R^2$ and $R^3$ are identical or different and each represents alkyl which has 1 to 12 carbon atoms and is optionally substituted by alkoxy having 1 to 4 carbon atoms, hydroxyl, halogen or N-methyl-N-benzylamino, and

$R^4$ represents cyano or alkyl which has 1 to 4 carbon atoms and is optionally substituted by hydroxyl or halogen, for treating depression, characterised in that the dihydropyridines are converted, using customary auxiliaries and vehicles, into a suitable administration form.

**Revendications**

**Revendications pour les Etats Contractants : AT, BE, CH, DE, FR, GB, IT, LI, NL, SE.**

1. Utilisation de dihydropyridines actives comme antagonistes du calcium, de formule générale :

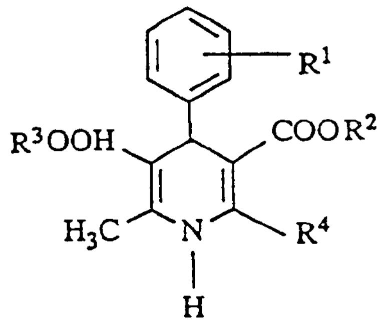

(I)

dans laquelle

$R^1$ représente un ou deux substituants identiques ou différents choisis parmi les halogènes et les groupes nitro, trifluorométhyle et $OCHF_2$ ou le reste =N-O-N=, qui est accolé au noyau phényle,

$R^2$ et $R^3$ sont identiques ou différents et représentent chacun un groupe alkyle en $C_1$-$C_{12}$, qui est éventuellement substitué par un atome d'halogène ou un groupe alcoxy en $C_1$-$C_4$, hydroxy ou N-méthyl, N-benzylamino et

$R^4$ représente un groupe cyano ou alkyle en $C_1$-$C_4$, qui peut être éventuellement substitué par un halogène ou un groupe hydroxy, pour la fabrication de médicaments pour le traitement des dépressions.

2. Préparations combinées antidépressives douées d'activité synergique, contenant une substance active antidépressive du groupe Nortriptylin, Amitriptylin, Imipramin, Desipramin, Doxepin, Citalopram, Fluoxetin, Fluovaxamin, Mianserin, Oxaprotilin et Maprotilin et une dihydropyridine de formule (I) selon la revendication 1.

3. Procédé pour la fabrication de préparations combinées antidépressives douées d'activité synergique, contenant une substance active antidépressive du groupe Nortriptylin, Amitriptylin, Imipramin, Desipramin, Doxepin, Citalopram, Fluoxetin, Fluovaxamin, Mianserin, Oxaprotilin et Maprotilin et une dihydropyridine active comme antagoniste du calcium, de formule générale

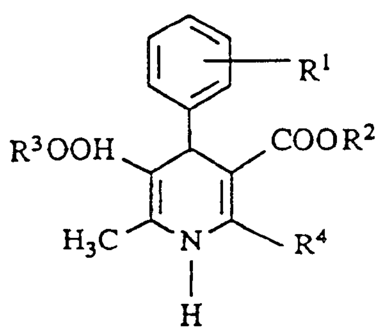

(I)

dans laquelle

$R^1$ représente un ou deux substituants identiques ou différents choisis parmi les halogènes et les groupes nitro, trifluorométhyle et $OCHF_2$ ou le reste =N-O-N=, qui est accolé au noyau phényle,

$R^2$ et $R^3$ sont identiques ou différents et représentent chacun un groupe alkyle en $C_1$-$C_{12}$, qui est éventuellement substitué par un atome d'halogène ou un groupe alcoxy en $C_1$-$C_4$, hydroxy ou N-méthyl, N-benzylamino et

$R^4$ représente un groupe cyano ou alkyle en $C_1$-$C_4$, qui peut être éventuellement substitué par un halogène ou un groupe hydroxy, caractérisé en ce que l'on transforme les dihydropyridines et les substances actives antidépressives en une forme d'application appropriée, éventuellement avec utilisation d'agents auxiliaires et supports habituels.

**Revendications pour L'Etat Contractant : ES.**

1. Procédé pour la fabrication de préparations combinées antidépressives douées d'activité synergique, contenant une substance active antidépressive du groupe Nortriptylin, Amitriptylin, Imipramin, Desipramin, Doxepin, Citalopram, Fluoxetin, Fluovaxamin, Mianserin, Oxaprotilin et Maprotilin et une dihydropyridine active comme antagoniste du calcium, de formule générale

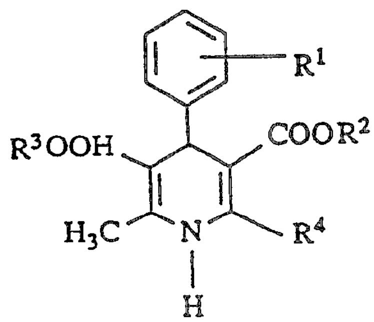

(I)

dans laquelle

$R^1$ représente un ou deux substituants identiques ou différents choisis parmi les halogènes et les groupes nitro, trifluorométhyle et $OCHF_2$ ou le reste =N-O-N=, qui est accolé au noyau phényle,

$R^2$ et $R^3$ sont identiques ou différents et représentent chacun un groupe alkyle en $C_1$-$C_{12}$, qui est éventuellement substitué par un atome d'halogène ou un groupe alcoxy en $C_1$-$C_4$, hydroxy ou N-méthyl, N-benzylamino et

$R^4$ représente un groupe cyano ou alkyle en $C_1$-$C_4$, qui peut être éventuellement substitué par un halogène ou un groupe hydroxy, caractérisé en ce que l'on transforme les dihydropyridines et les substances actives antidépressives en une forme d'application appropriée, éventuellement avec utilisation d'agents auxiliaires et supports habituels.

2. Procédé selon la revendication 1, caractérisé en ce que l'on utilise comme dihydropyridines antagonistes du calcium le Nifedipin, le Niludipin, le Nisoldipin, le Nitrendipin, le Nimodipin, le Felodipin ou le Nicardipin.

3. Procédé selon la revendication 1 dans lequel la substance antidépressive est présente dans le médicament en quantité de 10 a 200 mg et la dihydropyridine active comme antagoniste du calcium en quantité de 5 à 100 mg.

4. Procédé pour la fabrication de médicaments contenant des dihydropyridines antagonistes du calcium de formule générale

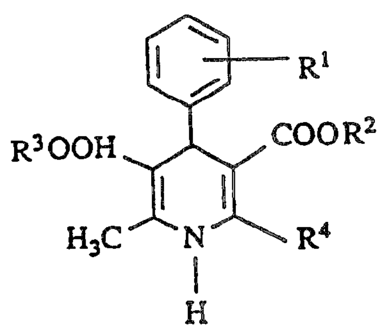

(I)

dans laquelle

$R^1$ représente un ou deux substituants identiques ou différents choisis parmi les halogènes et les groupes nitro, trifluorométhyle et $OCHF_2$ ou le reste =N-O-N=, qui est accolé au noyau phényle,

$R^2$ et $R^3$ sont identiques ou différents et représentent chacun un groupe alkyle en $C_1$-$C_{12}$, qui est éventuellement substitué par un atome d'halogène ou un groupe alcoxy en $C_1$-$C_4$, hydroxy ou N-méthyl, N-benzylamino et

$R^4$ représente un groupe cyano ou alkyle en $C_1$-$C_4$, qui peut être éventuellement substitué par un halogène ou un groupe hydroxy, pour le traitement des dépressions, caractérisé en ce que l'on transforme les dihydropyridines en une forme d'application appropriée en utilisant des substances auxiliaires et supports habi-

tuels.